(19) Europäisches Patentamt
European Patent Office
Office européen des brevets

(11) Publication number: **0 156 224**
**B1**

(12) **EUROPEAN PATENT SPECIFICATION**

(45) Date of publication of patent specification: **28.11.90**

(51) Int. Cl.$^5$: **C 07 D 327/06,** A 61 K 31/39

(21) Application number: **85102644.3**

(22) Date of filing: **08.03.85**

(54) **Novel benzoxathiin derivative, process for preparing them and pharmaceutical composition.**

(30) Priority: **08.03.84 JP 44730/84**

(43) Date of publication of application:
**02.10.85 Bulletin 85/40**

(45) Publication of the grant of the patent:
**28.11.90 Bulletin 90/48**

(84) Designated Contracting States:
**AT BE CH DE FR GB IT LI LU NL SE**

(56) References cited:

**CHEMICAL ABSTRACTS, vol. 100, no. 15, 9th April 1984, page 596, abstract no. 121093z, Columbus, Ohio, US; & JP - A - 58 170 778 (SUNTRY LTD.) 07.10.1983**

**JOURNAL OF MEDICINAL CHEMISTRY, vol. 15, no. 3, March 1972, pages 260-266; F. CROWTHER et al.: "Beta-Adrenergic blocking agents. 12. Heterocyclic compounds related to propranolol"**
**Burger s Medicinal Chemistry, 4th edition, vol. II, pages 84, 87.**

(73) Proprietor: **Suntory Limited**
**1-40 Dojimahama 2-chome Kita-ku**
**Osaka-shi Osaka-fu 530 (JP)**

(72) Inventor: **Hori, Mikio**
**33-1, Honmachi 5-chome**
**Gifu-shi Gifu-ken (JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81 (DE)**

**EP 0 156 224 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a benzoxathiin derivative represented by the general formula

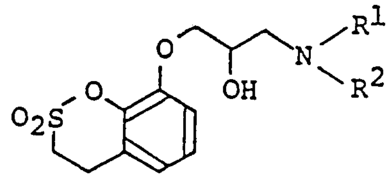

(1)

wherein $R^1$ represents a hydrogen atom or a $C_1$—$C_5$ alkyl group, and $R^2$ represents a hydrogen atom, a $C_1$—$C_5$ alkyl group or a group represented by $-(CH_2)_n-$Ar, wherein n is 1 or 2, and Ar is an unsubstituted phenyl group or a phenyl group substituted with at least one lower alkoxy group, or a pharmaceutically acceptable acid addition salt thereof.

This invention also relates to the production process of the benzoxathiin derivative represented by the general formula (1) and to pharmaceutical compositions, preferably to combat diseases of the cardiovascular system.

Since pronethalol emerged for the first time in 1962, a number of β-adrenaline receptor blocking agents such as propranolol and the like have been developed.

Chemical Abstracts, vol. 100, no. 15 page 596, abstract no. 121093z discloses the use of various benzoxathiin derivatives having a side chain at the 5 position as medicaments having β-blocking activity.

The Journal of Medicinal Chemistry, vol. 15, no. 3 (March 1972), pp. 260—264 discloses that various heterocyclic systems based on propranolol have improved β-blocking activity when a side chain is attached to the benzenoid part of the structure at the α position. However, none of the heterocyclic systems exemplified is at all similar to the benzoxathiin derivatives of the present invention.

The β-blockers reveal their activities by antagonizing the β-action of catecholamines released from the peripheries of sympathetic nerve or adrenal medulla. The clinical effectiveness of these β-blockers for arrythmia is considered to be due first of all to the blocking of the β-receptor and also to the participation of the quinidine-like actions. The effects of the β-blockers on angina pectoris are supposedly due to the blocking of catecholamine action, theeby promoting a reduction of cardiac hyperergasia against constant motion loads, and the β-blockers thus act defensively.

The effects of the β-blockers on hypertension, though still remaining ambiguous, are supposedly based on the suppression of the release of renin, the suppression of the cardiac muscle, the control of the central autonomic nerve and the like.

Thus, the β-blockers arre now widely used as remedies for arrhythmia, angina pectoris and hypertension.

During the course of research which was conducted in order to develop a β-blocker superior to the currently available β-blockers in terms of such points as the ability to block the β-adrenaline receptor, the membrane stabilizing effect (local anesthetic and quinidine-like action) or the like, the present inventors have found a novel benzoxathiin derivative having low toxicity and side-effects and a long duration, and have thereby accomplished his invention.

According to this invention, there are provided benzoxathiin derivatives having said general formula (1) or pharmaceutically acceptable acid addition salts thereof, a production process for the same and their use as a remedy for diseases of the cardiovascular system such as the treatment of arrhythmia, angina pectoris and hypertension.

Typical compounds represented by the general formula according to this invention include the following:

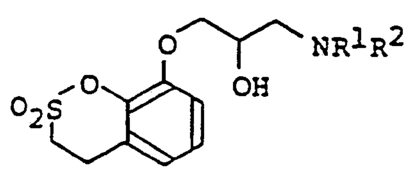

(1)

wherein —$NR^1R^2$ represents amino group, methylamino group, ethylamino group, n-propylamino group, isopropylamino group, n-butylamino group, isobutylamino group, sec-butylamino group, t-butylamino group, n-pentylamino group, isopentylamino group, neopentylamino group, t-pentylamino group, benzylamino group, phenethylamino group, 4-methoxyphenethylamino group, 2,3-dimethoxyphenethylamino group, 3,4-dimethoxyphenethylamino group, dimethylamino group, ethylmethylamino group, methylpropylamino group, isopropylmethylamino group, butylmethylamino group, isobutylmethylamino group, sec-butylmethylamino group, t-butylmethylamino group, methylpentylamino group, isopentylmethylamino group, methylneopentylamino group, methyl-t-pentylamino group, benzylmethylamino

group, methylphenethylamino group, methyl-4-methoxyphenethylamino group, 2,3-dimethoxyphenethylmethylamino group, 2,3-dimethoxyphenethylmethylamino group and 3,4-dimethoxyphenethylmethylamino group.

The compounds (1) of this invention are basic compounds and form salts with pharmaceutically acceptable acids. Suitable acids in this case include, for example, mineral acids such as hydrogen chloride, hydrobromic acid, hydroiodic acid, phosphoric acid and sulfuric acid, and appropriate organic carboxylic acids or sulfonic acids such as trifluoroacetic acid, acetic acid, p-toluenesulfonic acid, benzenesulfonic acid, maleic acid, citric acid, oxalic acid, succinic acid, benzoic acid, tartaric acid, fumaric acid, mandelic acid, ascorbic acid and malic acid.

The compounds (1) of this invention are obtained by treating 8-(2,3-epoxypropoxy)-3,4-dihydro-1,2-benzoxathiin-2,2-dioxide (2) with various amino compounds.

The reaction is performed either at room temperature or by heating 8-(2,3-epoxypropoxy)-3,4-dihydro-1,2-benzoxathiin-2,2-dioxide (2) with an amine, for example, an alkyl amine such as aqueous ammonia, methylamine, ethylamine, propylamine, butylamine or pentylamine; a dialkylamine such as dimethylamine, diethylamine, ethylmethylamine, methylpropylamine, butylmethylamine or methylpentylamine; or an aralkylamine or a lower alkoxyaralkylamine such as benzylamine, benzylmethylamine, phenethylamine, methylphenethylamine, 4-methoxyphenethylamine, 4-methoxyphenethylmethylamine, 2,3-methoxyphenethylamine, 2,3-methoxyphenethylmethylamine, 3,4-methoxyphenethylamine or 3,4-methoxyphenethylmethylamine.

The solvent used is preferably water or an alcohol.

The compounds thus obtained form corresponding salts by treating with pharmaceutically acceptable acids.

The above-mentioned 8-(2,3-epoxypropoxy)-3,4-dihydro-1,2-benzoxathiin-2,2-dioxide (2) is a novel compound and can be produced from o-vanillin as a raw material. o-Vanillin and methanesulfonyl chloride are condensed in the presence of a base to form 3-methoxy-2-methanesulfonyloxybenzaldehyde (3). The base used is preferably trimethylamine.

3-Methoxy-2-methanesulfonyloxybenzaldehyde (3) is cyclized by a base, for example, a strong base such as potassium hydroxide or sodium hydroxide to form 4-hydroxy-8-methoxy-3,4-dihydro-1,2-benzoxathiin-2,2-dioxide (4).

This compound (4), on treating with a dehydrating agent such as thionyl chloride or phosphorous oxychloride, forms a dehydrated product 8-methoxy-1,2-benzoxathiin-2,2-dioxide (5).

When 8-methoxy-1,2-benzoxathiin-2,2-dioxide (5) is reduced with a catalyst, 8-methoxy-3,4-dihydro-1,3-benzoxathiin-2,2-dioxide (6) which is a reduction product of the double bond of the compound (5) is obtained in a good yield.

As the catalyst used in this reduction, there can be mentioned Raney nickel, palladium on carbon, palladium and platinum.

Subsequently, 8-methoxy-3,4-dihydro-1,2-benzoxathiin-2,2-dioxide (6) is treated with hydrobromic acid, hydroiodic acid, aluminium chloride, hydrogen chloride, boron tribromide or the like to form 8-hydroxy-3,4-dihydro-1,2-benzoxathiin-2,2-dioxide (7).

When 8-hydroxy-3,4-dihydro-1,2-benzoxathiin-2,2-dioxide (7) thus obtained and epichlorohydrin are reacted by heating in the presence of a base, 8-(2',3'-epoxypropoxy)-3,4-dihydro-1,2-benzoxathiin-2,2-dioxide (2) is obtained as the raw material of this invention. The base used in this reaction is preferably potassium carbonate or sodium carbonate, lithium carbonate.

The above-mentioned processes are shown in the following reaction formulae:

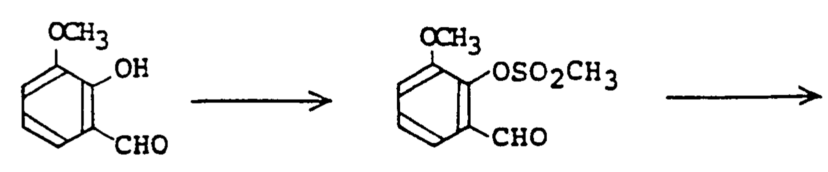

o-vanillin (3)

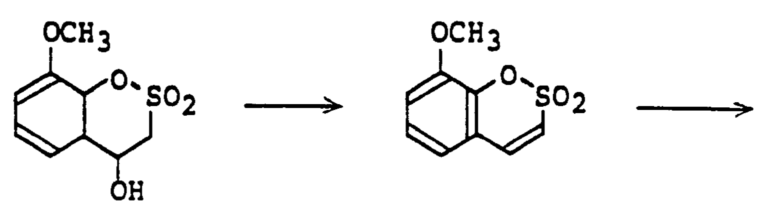

(4) (5)

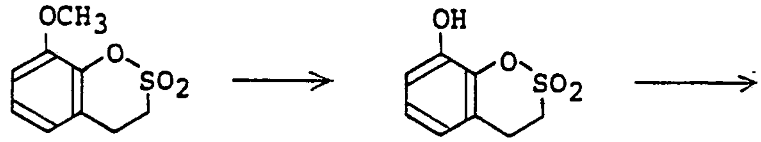

(6) (7)

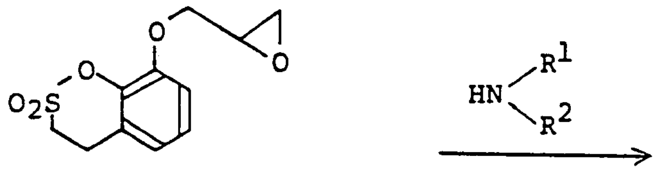

(2)

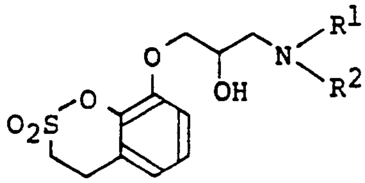

(1)

wherein $R^1$ and $R^2$ have the same meanings as defined above.

The test methods of the compounds of this invention for various pharmacological effects on the cardiovascular system and the results thereof are described below.

*Pharmacological Experiments*

(1) Test of the β-blocking effect

Preparation of a membrane specimen

Spraque-Dawley male rats having 150—200 g body weight were decapitated, and the brains were quickly taken out and, after decortication, frozen at −70°C. After freezing overnight at −70°C, the thawed brain was homogenized with a buffer [0.25 mole sucrose, 5 mmole tris(hydroxymethyl)aminomethane hydrochloride, 1 mmole magnesium chloride, pH 7.4] in an amount of 8 times the weight of the brain. The homogenate was centrifuged at 800 × g for 20 minutes, and the supernatant was centrifuged at 14,000 × g for 14 minutes, the deposit of which was washed three times with the above-mentioned buffer. The deposit finally obtained was suspended again in a buffer of 75 mmole tris(hydroxymethyl)aminomethane and 25 mmole magnesium chloride (pH 7.4) to form a membrane specimen.

Test method

To 50 l of the membrane specimen were added 50 l of $^3$H-dihydroalprenolol ($^3$H-OHA) and 50 l of the mixture of the test frug and the buffer, such that the total volume was adjusted to 150 l, and the mixture was incubated at 37°C for 10 minutes.

The reaction was stopped by adding an ice-cooled mixture of 50 mmole tris(hydroxymethyl)aminomethane hydrochloride and 17 mmole magnesium chloride (pH 7.4), and the mixture was filtered under reduced pressure through a Whatman GF/B glassfiber. The filter was washed three times and dried, and a toluene type scintillator was added to measure the radioactivity.

In the measurement, the amount obtained by subtracting the bondings obtained in the presence of $10^{-5}$ mole propanolol from the total bondings was considered to represent the specific bondings of the β-receptor. In addition, the determination of protein was carried out according to the method of Lowry et al. [Lowry, O. H. et al., J. Biol. Chem., *193*, 265—275 (1951)]. The results are shown in Table 1.

Table 1 Test Result

β-Receptor blocking effect of the synaptosome membrane of rat brain

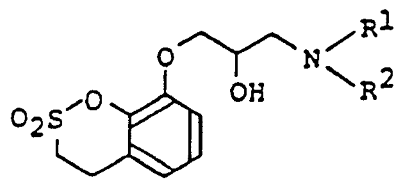

| Example code of test compound | 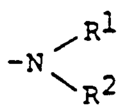 | β-Receptor blocking effect Inhibition (%) | |
|---|---|---|---|
| | | $10^{-6}$ mole | $10^{-5}$ mole |
| 1 | methylamino group, fumarate | 35.4 | 73.9 |
| 2 | dimethylamino group, fumarate | 17.1 | 62.6 |
| 3 | isopropylamino group, hydrochloride | 97.6 | 91.6 |
| 4 | t-butylamino group, hydrochloride | 96.3 | 91.4 |
| 5 | sec-butylamino group, hydrochloride | 83.0 | 95.3 |
| 6 | isobutylamino group, hydrochloride | 41.7 | 81.9 |
| 7 | 3 4 -dimethoxyphenethyl group, hydrochloride | 75.2 | 90.1 |

(2) Test of anti-arrhythmic effect

Referring to the method described by J. W. Lawson (Journal of Pharmacology and Experimental Therapeutics, *160,* 22, 1968), a ddy male mouse was subjected to the inhalation of chloroform, and when respiration stopped, an electrocardiogram was recorded and the ventricular flutter and fibrillation were observed. When a compound showing an anti-arrhythmic effect is previously administered, such an irregular ventricular appearance is prevented. The compound of this invention was intraperitoneally administered in various dosages to the groups of 29—40 mice. After 30 minutes the mice were subjected to the inhalation of chloroform, and the preventive ratios of the ventricular flutter and fibrillation were measured for respective dosages.

Then, according to the method of Litchfield and Wilcoxon (Journal of Pharmacology and Experimental Therapeutics, *96,* 99, 1949), the 50% effective dose ($ED_{50}$) and 95% confidence limit thereof were calculated and shown in Table 3.

As is apparent from Table 3, the compounds of this invention have an excellent anti-arrhythmic effect in half of the amount or even in an extremely low dosage as compared to the conventional compounds, and showed a property of retaining the anti-arrhythmic effect even after 24 hours.

(3) Effects on the blood pressure and heart rate in SHR

In order to measure the blood pressure of a SHR (spontaneous hypertensive rat), a polyethylene tube was inserted into an artery to a level of the abdominal aorta through the crural artery, and the other end of the tube was tightly stoppered by a metal rod, extended subcutaneously and exposed out of the body at the posterior of the neck and fixed by suturation to the surrounding skin. The tube was filled with 500 U of heparin.

On the day subsequent to the operation, the blood pressure was measured without anesthetization or restriction using a pressure transducer and continuously recorded on a thermal recording system together with the heart rate which was measured by driving a tachometer with the pulse wave of blood pressure.

The test drugs were dissolved in physiological saline and administered using a sound (p.o.).

Before administration of drugs the blood pressures and heart rates of SHR showed no significant difference as between the control group, to which the physiological saline was administered, and groups treated with SUN3479 (the product in Example 4) or carteolol.

The blood pressure and heart rate increased only slightly, probably as a result of being handled during the administration of the physiological saline (5 ml/kg), from the time immediately after the administration until 1 hour after the administration, but thereafter they started to show the tendency to decrease.

The changes of blood pressure with the passage of time after the administration of drugs in the three groups is shown in Table 2.

Table 2 Effects of product of Example 4 (SUN 3479) and carteolol on mean blood pressure in conscious spontaneously hypertensive rats.

| | Time | Control | SUN 3479 | | Carteolol | |
|---|---|---|---|---|---|---|
| | | (saline) (n=4) | 0.1 mg/kg p.o. (n=4) | 1 mg/kg p.o. (n=5) | 0.1 mg/kg p.o. (n=4) | 1 mg/kg p.o. (n=5) |
| Basal value (mmHg) | | 145.2 ± 3.2 | 154.5 ± 14.6 | 166.4 ± 4.5 | 149.0 ± 7.9 | 159.0 ± 6.7 |
| % Exchange | 10 min | 4.1 ± 2.3 | 6.4 ± 3.5 | 1.4 ± 1.2 | 1.4 ± 2.1 | 2.5 ± 3.2 |
| | 20 min | 3.1 ± 1.3 | 4.6 ± 3.2 | -0.3 ± 1.7 | -9.2 ± 4.0 | -1.4 ± 2.6 |
| | 30 min | 2.0 ± 1.3 | 4.1 ± 2.6 | -8.1 ± 2.9* | -11.3 ± 3.9* | -5.1 ± 3.6 |
| | 60 min | -0.3 ± 1.5 | -4.2 ± 3.8 | -12.0 ± 4.3* | -.7.3 ± 2.7* | -11.7 ± 3.3** |
| | 90 min | -4.2 ± 2.8 | -6.5 ± 3.2 | -10.9 ± 2.8** | -18.1 ± 3.7** | -14.7 ± 3.6** |
| | 120 min | -6.3 ± 3.4 | -7.3 ± 5.9 | -14.5 ± 4.3** | -12.7 ± 6.5 | -23.8 ± 3.0*** |
| | 3 hr | -5.8 ± 3.3 | -8.9 ± 3.0 | -16.3 ± 4.1** | -15.4 ± 3.3** | -23.1 ± 4.8** |
| | 4 hr | -3.9 ± 5.0 | -13.0 ± 2.5** | -20.0 ± 3.7*** | -15.1 ± 3.7** | -24.4 ± 4.2*** |
| | 5 hr | -7.8 ± 0.8 | -14.7 ± 2.5*** | -24.5 ± 4.0*** | -18.8 ± 1.3*** | -24.7 ± 4.7*** |
| | 6 hr | -6.7 ± 3.8 | -14.8 ± 2.4*** | -24.1 ± 3.3*** | -20.8 ± 1.7*** | -21.3 ± 4.2*** |
| | 24 hr | | -2.8 ± 4.2 | -11.5 ± 6.2 | -2.1 ± 2.5 | -2.1 ± 4.5 |

Each value represents the mean ±SEM.

Significantly different from control,

* p<0.05

** p<0.01

*** p<0.001

As apparent from Table 2, the compound of Example 4 has an excellent hypotensive effect at almost the same level as carteolol, and it remains effective even after 24 hours with no significant difference being recognized.

(4) Acute toxicity

The test compounds were administered in the caudal rein or stomach of ddy male mice having 18—22 g body weight, and the $LD_{50}$ was calculated from the mortality occurring within 1 week after administration, using the up and down method (Edited by Takagi and Ozawa; YAKUBUTSUGAKU JIKKEN, p. 204, Nanzando, 1972). The results are shown in Table 3.

Table 3 Anti-arrhythmic effect and acute toxicity of the compounds of this invention

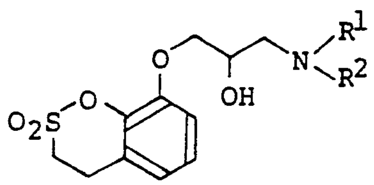

| Example code of test compound | 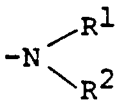 | Anti-arrhythmic effect ($ED_{50}$) mg/kg | | Acute toxicity ($LD_{50}$) mg/kg | |
|---|---|---|---|---|---|
| | | $ED_{50}$ | 95% confidence limit | intra-venous | in stomach |
| 3 | isopropylamino group, hydrochloride | 0.23 | 0.10 - 0.54 | 68.2 | >500 |
| 4 | t-butylamino group, hydrochloride | 0.03 | 0.01 - 0.07 | 59.2 | >500 |
| 5 | sec-butylamino group, hydrochloride | 1.30 | 0.45 - 3.72 | 68.2 | >500 |
| | propranolol | 2.20 | 1.03 - 4.70 | 21.9 | - |
| | carteolol | 400.00 | 195.89 - 816.00 | 38.0 | - |
| | acebutolol | 47.00 | 20.80 - 106.22 | 40.2 | - |

As is apparent from Table 3, the compounds of this invention show very low toxicities in both intravenous and oral administrations. Particularly in oral administration, there was no mortality even at a dose of 500 mg/kg.

The compounds of this invention, which are effective β-blockers, may be applied to treat angina pectoris and hypertension in addition to arrhythmia.

The compounds of this invention are used in the form of the free bases, or in the form of pharmaceutically acceptable salts such as the hydrochloride or the fumarate, as a remedy for arrhythmia. They may be administered orally or parenterally, alone or in combination with known non-toxic vehicles in an appropriate dosage form such as capsule, tablet or injection, and these preparations may be prepared, for example, as follows. After the raw material is pulverized, it is mixed with a vehicle such as lactose, starch or a derivative thereof or a cellulose derivative and is then charged into a gelatin capsule to form a capsule. In order to make a tablet, a bonding agent such as carboxymethylcellulose sodium, arginic acid or gum arabic

and water are added in addition to the above-mentioned vehicle, mixed together and granulated with an extrusion granulator. To the granules obtained is added a lubricant such as talc or stearic acid, and tablets are prepared with a usual suppression tablet machine. For parenteral administration by injection, a watersoluble salt of the compound is dissolved in sterilized distilled water or sterilized physiological saline and charged in an ampule to provide a preparation for injection. It may include a stabilizer and/or a buffer substance, if necessary.

The effective amount of anti-arrhythmic agent varies depending on the kind and strength of arrhythmia or the physical condition of a patient, but it is usually dosed in an amount sufficient to make an irregular rhythm return to a sinus rhythm. The compounds of this invention may be orally administered in an amount of 10—30 mg per dosage for an adult, 2—3 times per day.

This invention is specifically explained by referring to the following Examples, but it will be understood that this invention is not limited thereto.

Example 1

8-(2-hydroxy-3-methylaminopropoxy)-3,4-dihydro-1,2-benzoxathiin-2,2-dioxide

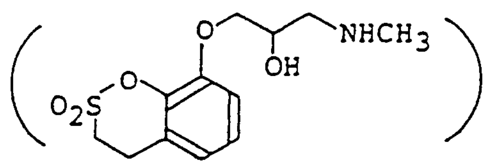

To 2.710 g (10.6 mmole) of 8-(2,3-epoxypropoxy)-3,4-dihydro-1,2-benzoxathiin-2,2-dioxide was added 60 ml of 30% methanolic solution of methylamine, and the mixture was stirred for 30 minutes at room temperature. Methylamine and methanol were removed by distillation under reduced pressure, and the residue was acidified by the addition of hydrochloric acid. The solution was extracted with chloroform, and the disused materials (including the raw material) were removed. Then, the hydrochloric acid layer was basified by the addition of dilute aqueous ammonia. The basic aqueous layer was extracted with chloroform (× 3), the extracts were dried over magnesium sulfate, and thereafter the solvent was removed by distillation under reduced pressure. To 1.95 g of the residue was aded 0.39 g of fumaric acid, and the mixture was dissolved in methanol and then recrystallized to obtain quantitatively the fumaric acid salt of the title compound.

Data of the fumarate

Appearance: colorless prismatic crystal

Melting point: 133.5—137°C

IR spectrum (KBr, $cm^{-1}$): 3400, 3080, 1590, 1380, 1160

Mass spectrum (m/z): 288 ($M^+$+1), 243 (b.p.)

NMR spectrum (DMSO-$d_6$, δ): 2.70—4.30 (12H, m), 5.40—6.10 (3H, m, *OH*, $N^+H_2$), 6.40 (1H, s, fumaric acid), 6.60—7.20 (3H, m)

| Elementary analysis: | C (%) | H (%) | N (%) |
|---|---|---|---|
| Calculated for | | | |
| $C_{12}H_{17}NO_5S \cdot \frac{1}{2}C_4H_4O_4 \cdot \frac{1}{2}H_2O$: | 47.45 | 5.69 | 3.95 |
| Found: | 47.59 | 5.77 | 3.86 |

Example 2

8-(3-dimethylamino-2-hydroxypropoxy)-3,4-dihydro-1,2-benzoxathiin-2,2-dioxide

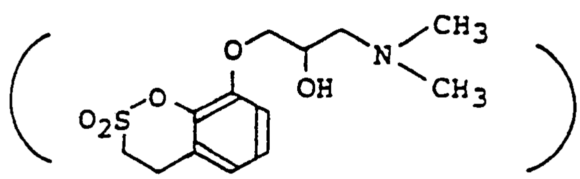

To 2.312 g (9.0 mmole) of 8-(2,3-epoxypropoxy)-3,4-dihydro-1,2-benzoxathiin-2,2-dioxide were added 30 ml of 50% aqueous dimethylamine solution and 30 ml of methanol, and the mixture was stirred for 30 minutes at room temperature. Treating in the same manner as in Example 1, 1.76 g (yield, 64%) of the title compound were obtained.

To this compound was added 0.33 g of fumaric acid, and the mixture was dissolved in methanol and recrystallized to obtain 1.5 g of the fumarate.

Data of the fumarate

Appearance: colorless crystal

Melting point: 144.9—145.7°C

| Elementary analysis: | C (%) | H (%) | N (%) |
|---|---|---|---|
| Calculated for $C_{13}H_{19}NO_5S\cdot\frac{1}{2}C_4H_4O_4$: | 50.13 | 5.89 | 3.90 |
| Found: | 49.85 | 5.93 | 3.87 |

IR spectrum (KBr, $cm^{-1}$): 3200, 1590, 1360, 1155

Mass spectrum (m/z): 302 ($M^+$+1), 257 (b.p.)

NMR spectrum (DMSO-$d_6$, δ): 2.40 (6H, s), 2.50—4.20 (9H, m), 6.45—6.80 (2H, m, O*H*, $N^+$*H*), 6.40—7.20 (3H, m).

Example 3

8-(2-hydroxy-3-isopropylaminopropoxy)-3,4-dihydro-1,2-benzoxathiin-2,2-dioxide

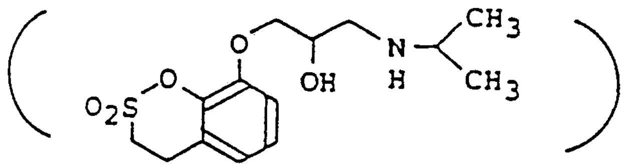

To 2.008 g (7.8 mmole) of 8-(2,3-epoxypropoxy)-3,4-dihydro-1,2-benzoxathiin-2,2-dioxide were added 10 ml of isopropylamine and 50 ml of methanol, and the mixture was refluxed by heating for 1 hour. Proceeding in the same manner as in Example 1, the reaction residue was obtained. The residue obtained was dissolved in chloroform and reacted with a hydrochloric acid gas saturated ether to form a hydrochloride. The salt was recrystallized from a mixed solvent of isopropanol and ether, and 1.28 g of the hydrochloride of the title compound was obtained (yield, 57%).

Data of the hydrochloride

Appearance: colorless prismatic crystal

Melting point: 168.0—170.0°C

| Elementary analysis: | C (%) | H (%) | N (%) |
|---|---|---|---|
| Calculated for $C_{14}H_{21}NO_5S\cdot HCl$: | 47.79 | 6.02 | 3.98 |
| Found: | 47.40 | 6.28 | 4.02 |

IR spectrum (KBr, $cm^{-1}$): 3370, 2950, 1365, 1165

Mass spectrum (m/z): 316 ($M^+$+1), 136 (b.p.)

NMR spectrum ($CDCl_3$, δ): 1.11 (6H, d, J—6.75Hz), 2.50—3.10 (3H, m), 3.00 (2H, s, O*H*, N*H*), 3.80—4.30 (3H, m), 6.70—7.30 (3H, m).

Example 4

8-(3-t-butylamino-2-hydroxypropoxy)-3,4-dihydro-1,2-benzoxathiin-2,2-dioxide

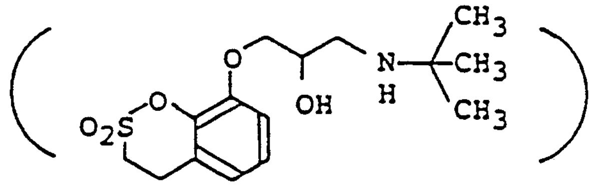

To 2.04 g (8.0 mmole) of 8-(2,3-epoxypropoxy)-3,4-dihydro-1,2-benzoxathiin-2,2-dioxide were added 10 ml of t-butylamine and 50 ml of methanol, and the mixture was refluxed by heating for 1 hour. The reaction mixture was treated in the same manner as in Example 1, and a reaction residue was obtained. The residue obtained was reacted with a hydrochloric gas saturated ether to form a hydrochloride. The hydrochloride obtained was recrystallized from a mixed solvent of methanol and ether and 1.52 g (yield, 52%) of the hydrochloride of the title compound was obtained.

Data of the hydrochloride

Appearance: colorless prismatic crystal

Melting point: 248.0—250.0°C

| Elementary analysis: | C (%) | H (%) | N (%) |
|---|---|---|---|
| Calculated for $C_{15}H_{23}NO_5S \cdot HCl$: | 49.24 | 6.61 | 3.83 |
| Found: | 49.05 | 6.62 | 3.80 |

IR spectrum (KBr, $cm^{-1}$): 3320, 2975, 1380, 1160

Mass spectrum (m/z): 330 ($M^+$+1), 314 (b.p.)

NMR spectrum ($CDCl_3$, δ): 1.14 (9H, s), 2.70—2.95 (2H, m), 3.24 (2H, s, O*H*, N*H*), 3.50 (4H, s), 4.07 (3H, m), 6.70—7.30 (3H, m).

Example 5

8-(3-sec-butylamino-2-hydroxypropoxy)-3,4-dihydro-1,2-benzoxathiin-2,2-dioxide

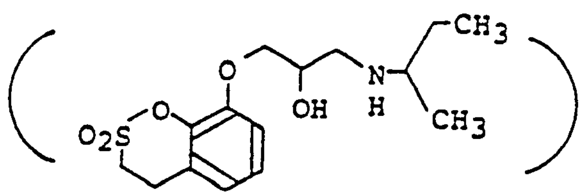

To 2.47 g (9.6 mmole) of 8-(2,3-epoxypropoxy)-3,4-dihydro-1,2-benzoxathiin-2,2-dioxide were added 10 ml of sec-butylamine and 50 ml of methanol, and the mixture was refluxed by heating for 30 minutes. The reaction mixture was treated in the same manner as in Example 1, whereby the reaction residue was obtained. The residue obtained was dissolved in chloroform and treated with a hydrochloric acid gas saturated ether to form a hydrochloride. Then, the salt was recrystallized from a mixed solvent of methanol and isopropanol and 1.54 g of the hydrochloride of the title compound was obtained (yield, 44%).

Data of the hydrochloride

Appearance: colorless prismatic crystals

Melting point: 169.5—170.0°C

| Elementary analysis: | C (%) | H (%) | N (%) |
|---|---|---|---|
| Calculated for $C_{15}H_{23}NO_5S \cdot HCl$: | 49.24 | 6.61 | 3.83 |
| Found: | 49.11 | 6.73 | 3.75 |

IR spectrum (KBr, $cm^{-1}$): 3320, 2960, 1370, 1160

Mass spectrum (m/z): 330 ($M^+$+1)

NMR spectrum ($CDCl_3$, δ): 0.70—1.80 (8H, m), 2.20—3.00 (3H, m), 2.68 (2H, m, O*H*, N*H*), 3.47 (4H, s), 4.05 (3H, m), 6.70—7.20 (3H, m).

Example 6

8-(2-hydroxy-3-isobutylaminopropoxy)-3,4-dihydro-1,2-benzoxathiin-2,2-dioxide

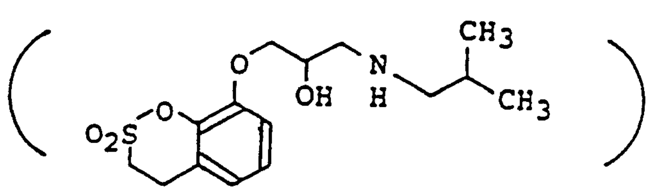

To 2.42 g (9.4 mmole) of 8-(2,3-epoxypropoxy)-3,4-dihydro-1,2-benzoxathiin-2,2-dioxide were added 10 ml of isobutylamine and 50 ml of methanol, and the mixture was refluxed by heating for 1.5 hours. The hydrochloride obtained by proceeding in the same manner as in Examples 1 and 3 was recrystallized from a mixed solvent of isopropanol and ether, and the hydrochloride of the title compound was obtained in an amount of 1.80 g (yield, 52%).

Data of the hydrochloride

Appearance: colorless prismatic crystals

Melting point: 140.0—140.5°C

| Elementary analysis: | C (%) | H (%) | N (%) |
|---|---|---|---|
| Calculated for $C_{15}H_{23}NO_5S \cdot HCl$: | 49.24 | 6.61 | 3.83 |
| Found: | 49.11 | 6.73 | 3.76 |

IR spectrum (KBr, $cm^{-1}$): 3280, 2960, 1368, 1158

Mass spectrum (m/z): 330 ($M^+$+1), 286 (b.p.)

NMR spectrum ($CDCl_3$, δ): 0.94 (6H, d, J—6.75Hz), 1.40—2.10 (1H, m), 2.38—2.95 (4H, m), 3.48 (4H, s), 3.95—4.28 (3H, m), 6.70—7.20 (3H, m).

## Example 7

8-[3-(3,4-dimethoxyphenethylamino)-2-hydroxypropoxy]-3,4-dihydro-1,2-benzoxathiin-2,2-dioxide

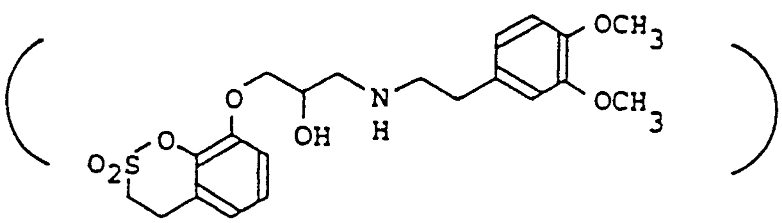

To 3.53 g (13.8 mmole) of 8-(2,3-epoxypropoxy)-3,4-dihydro-1,2-benzoxathiin-2,2-dioxide were added 10 ml of 3,4-dimethoxyphenethylamine and 50 ml of methanol, and the mixture was refluxed by heating for 20 hours. After methanol and most of the 3,4-dimethoxyphenethylamine had been removed by distillation under reduced pressure, the residue was treated in the same manner as in Example 3 to form a hydrochloride. The hydrochloride was dissolved in methylene chloride, and isopropanol was added. Methylene chloride was removed by distillation, and the precipitated powder was collected by filtration and recrystallized from methylene chloride to obtain the hydrochloride of the title compound in an amount of 1.24 g (yield, 20%).

Data of the hydrochloride

Appearance: colorless prismatic crystals

Melting point: 193.0—194.5°C

| Elementary analysis: | C (%) | H (%) | N (%) |
|---|---|---|---|
| Calculated for $C_{21}H_{27}NO_7S \cdot HCl \cdot \frac{1}{4}H_2O$: | 52.71 | 6.00 | 2.92 |
| Found: | 52.62 | 5.92 | 2.90 |

IR spectrum (KBr, $cm^{-1}$): 3300, 2960, 1370, 1160

Mass spectrum (m/z): 438 ($M^+$+1), 286 (b.p.)

NMR spectrum (DMSO-$d_6$, δ): 2.70—4.30 (13H, m), 3.35 (3H, m, O*H*, $N^+H_2$), 3.76 (3H, s), 3.80 (3H, s), 6.70—7.30 (6H, m).

## Preparation 1

2-mesyloxy-3-methoxybenzaldehyde

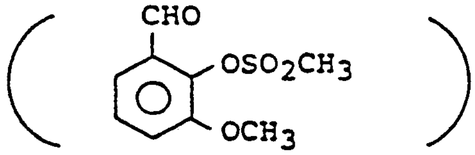

In 700 ml of acetone were dissolved 100 g (0.66 mole) of o-vanillin and 60 ml (0.78 mole) of methanesulfonyl chloride, and 200 ml (1.43 mole) of triethylamine was added dropwise with ice-cooling over a period of 2 hours.

Precipitated triethylamine hydrochloride was collected by filtration and washed further with 300 ml of acetone, and the combined filtrate was concentrated. The concentrated residue was dissolved in chloroform, washed with dilute aqueous hydrogen chloride and dried. Thereafter, chloroform was removed by distillation. By recrystallizing the residue from chloroform and ether, the title compound was obtained in an amount of 134.1 g (yield, 89%).

Appearance: colorless prismatic crystals
Melting point: 73.5—74.5°C

| Elementary analysis: | C (%) | H (%) |
|---|---|---|
| Calculated for $C_9H_{10}SO_5$: | 46.95 | 4.38 |
| Found: | 46.85 | 4.25 |

IR spectrum (KBr, $cm^{-1}$): 1690, 1360, 1280, 1150, 1060
Mass spectrum (m/z): 230 ($M^+$), 151 (b.p.)
NMR spectrum ($CDCl_3$, δ): 3.39 (3H, s), 4.00 (3H, s), 7.25—7.70 (3H, m), 10.38 (1H, s).

Preparation 2

4-hydroxy-8-methoxy-3,4-dihydro-1,2-benzoxathiin-2,2-dioxide

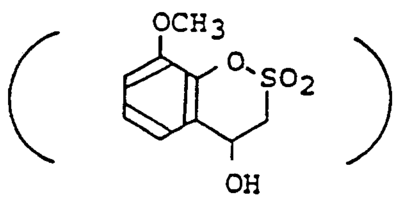

In 50 ml of pyridine was dissolved 8.00 g (34.7 mmole) of 2-mesyloxy-3-methoxybenzaldehyde, and 5 g of powdery potassium hydroxide was added to the solution with ice-cooling. The mixture was stirred for 1 hour. The reaction solution was poured into 5% aqueous hydrogen chloride and 300 g of crushed ice and extracted with ethyl acetate. The solvent was removed by distillation, and the residue was recrystallized from a mixed solvent of acetone and hexane. The title compound was obtained in an amount of 5.71 g (yield, 71%).

Appearance: colorless needles
Melting point: 108.5—110.0°C

| Elementary analysis: | C (%) | H (%) |
|---|---|---|
| Calculated for $C_9H_{10}SO_5$: | 46.95 | 4.38 |
| Found: | 46.74 | 4.34 |

IR spectrum (KBr, $cm^{-1}$): 3400, 1385, 1280, 1160, 1085
Mass spectrum (m/z): 230 ($M^+$), 212 (b.p.)
NMR spectrum (DMSO-$d_6$, δ): 3.42 (3H, s), 3.50—4.50 (3H, m, including O*H*), 4.95—5.35 (1H, m), 7.10—7.85 (3H, m).

Preparation 3

8-methoxy-1,2-benzoxathiin-2,2-dioxide

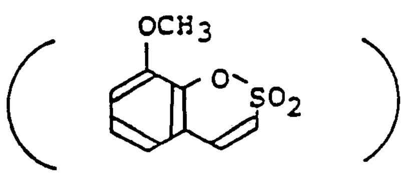

In 15 ml of pyridine was dissolved 3.35 g (14.6 mmole) of 4-hydroxy-8-methoxy-3,4-dihydro-1,2-benzoxathiin-2,2-dioxide, and 2.60 g (21.8 mmole) of thionyl chloride was added with ice-cooling to the solution. Furthermore, heating was conducted to complete the reaction. Pyridine was removed by distillation under reduced pressure, and the residue was dissolved in chloroform and washed with water. The chloroform layer was dried and thereafter concentrated, and the residue was subjected to silica gel column chromatography (eluent; benzene). The crystals obtained were recrystallized from methylene chloride-hexane, and the title compound was obtained in an amount of 2.21 g (yield, 72%).

Appearance: colorless prismatic crystals
Melting point: 130.5—131.7°C

| Elementary analysis: | C (%) | H (%) |
|---|---|---|
| Calculated for $C_9H_8SO_4$: | 50.94 | 3.80 |
| Found: | 51.08 | 3.82 |

IR spectrum (KBr, $cm^{-1}$): 1370, 1280, 1170, 1080
Mass spectrum (m/z): 212 ($M^+$)
NMR spectrum ($CDCl_3$, δ): 3.96 (3H, s), 6.80 (1H, d, J=10Hz), 6.90—7.25 (3H, m), 7.24 (1H, d, J=10Hz).

Preparation 4

8-methoxy-3,4-dihydro-1,2-benzoxathiin-2,2-dioxide

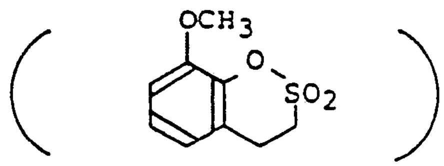

9.58 g (45.1 mmole) of 8-methoxy-1,2-benzoxathiin-2,2-dioxide and 2.0 g of palladium on carbon were suspended in 250 ml of methanol and 50 ml of dioxane, and the mixture was stirred at room temperature in the presence of hydrogen at an initial pressure of 100 atm for 3 days.

The catalyst was collected by filtration and washed with acetone, and the organic layer was then concentrated. The condensed residue was recrystallized from a mixed solvent of chloroform and methylene chloride; and the title compound was obtained in an amount of 8.96 g (yield, 93%).

Appearance: colorless needles

Melting point: 130.0—131.0°C

| Elementary analysis: | C (%) | H (%) |
|---|---|---|
| Calculated for $C_9H_{10}SO_4$: | 50.46 | 4.71 |
| Found: | 50.24 | 4.70 |

IR spectrum (KBr, $cm^{-1}$): 1370, 1270, 1150, 1080

Mass spectrum (m/z): 214 ($M^+$), 106 (b.p.)

Preparation 5

8-Hydroxy-3,4-dihydro-1,2-benzoxathiin-2,2-dioxide

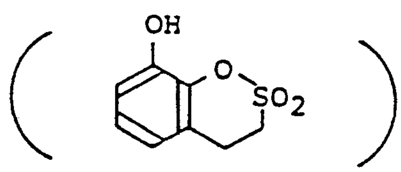

To 8.96 g (41.8 mmole) of 8-methoxy-3,4-dihydro-1,2-benzoxathiin-2,2-dioxide was added 100 ml of 47% hydrobromic acid, and the mixture was dissolved by heating and refluxed by heating for a further 1.5 hours.

After cooling the mixture, precipitated crystals were collected by filtration and washed with water. The aqueous solution was extracted with ether, and the ethereal extract was dried. Then, the solvent was removed by distillation, and crystals obtained were combined and dried (yield, quantitatively).

Appearance: colorless crystals

Melting point: 146.5—148.0°C

| Elementary analysis: | C (%) | H (%) |
|---|---|---|
| Calculated for $C_8H_8SO_4$: | 47.99 | 4.03 |
| Found: | 47.93 | 4.23 |

IR spectrum (KBr, $cm^{-1}$): 3520, 1375, 1150

Mass spectrum (m/z): 200 ($M^+$), 136 (b.p.)

Preparation 6

8-(2,3-epoxypropoxy)-3,4-dihydro-1,2-benzoxathiin-2,2-dioxide

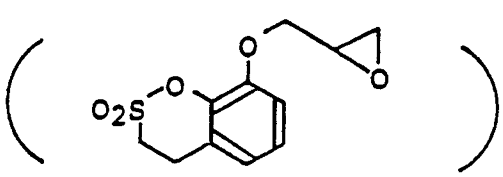

In 50 ml of acetone were dissolved 3.30 g (16.5 mmole) of 8-hydroxy-3,4-dihydro-1,2-benzoxathiin-2,2-dioxide and 6 ml (76 mmole) of epichlorohydrin, 3 g of potassium carbonate was added to the solution, and the mixture was heated under reflux for 38 hours. After removal of acetone by distillation, the residue was extracted with chloroform, and the extract was dried and concentrated under reduced pressure. The

residue was recrystallized from a mixed solvent of chloroform and hexane, and the title compound was obtained in an amount of 3.87 g (yield, 92%).

Appearance: colorless prismatic crystals

Melting point: 103.5—104.2°C

| Elementary analysis: | C (%) | H (%) |
|---|---|---|
| Calculated for $C_{11}H_{12}SO_5$: | 51.55 | 4.71 |
| Found: | 51.29 | 4.84 |

IR spectrum (KBr, $cm^{-1}$): 1370, 1280, 1160, 1080, 920, 770

Mass spectrum (m/z): 256 ($M^+$), 136 (b.p.)

NMR spectrum ($CDCl_3$, δ): 2.70—3.05 (2H, m), 3.20—3.50 (1H, m), 3.50 (4H, s), 3.88—4.50 (2H, m), 6.70—7.30 (3H, m).

## Claims

1. A benzoxathiin derivative having the general formula

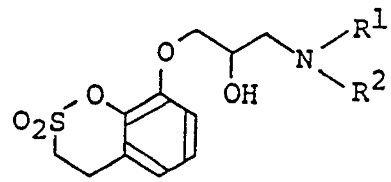

(1)

wherein $R^1$ represents a hydrogen atom or a $C_1$—$C_5$ alkyl group, and $R^2$ represents a hydrogen atom, a $C_1$—$C_5$ alkyl group or a group represented by $-(CH_2)_n-$Ar, wherein n is 1 or 2, and Ar is an unsubstituted phenyl group or a phenyl group substituted with at least one lower alkoxy group, or a pharmaceutically acceptable acid addition salt thereof.

2. A benzoxathiin derivative according to Claim 1, wherein $R^1$ represents a hydrogen atom or methyl group, and $R^2$ represents a methyl group, ethyl group, isopropyl group, isobutyl group, t-butyl group, sec-butyl group, n-propyl group or n-butyl group.

3. A benzoxathiin derivative according to Claim 1, wherein $R^1$ represents a hydrogen atom or methyl group, and $R^2$ represents a phenethyl group, 3,4-dimethoxyphenethyl group, 2,3-dimethoxyphenethyl group or 4-methoxyphenethyl group.

4. A pharmaceutical composition comprising a benzoxathiin derivative according to any preceding claim and a pharmaceutically acceptable carrier.

5. A benzoxathiin derivative according to any of claims 1—3 for use as a medicament.

6. A benzoxathiin derivative according to any of claims 1—3 for use in treating disorders of the cardiovascular system.

7. The use of a benzoxathiin derivative having the general formula

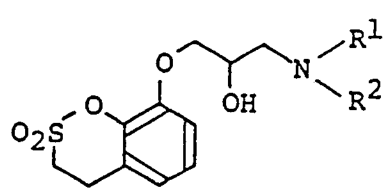

(1)

wherein $R^1$ represents a hydrogen atom or a $C_1$—$C_5$ alkyl group, and $R^2$ represents a hydrogen atom, a $C_1$—$C_5$ alkyl group or a group represented by $-(CH_2)_n-$Ar, wherein n is 1 or 2, and Ar is an unsubstituted phenyl group or a phenyl group substituted with at least one lower alkoxy group, or a pharmaceutically acceptable acid addition salt thereof for the manufacture of a medicament for the treatment of disorders of the cardiovascular system.

8. A process for producing a benzoxathiin derivative having the general formula

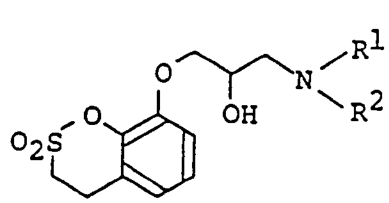

(1)

wherein $R^1$ represents a hydrogen atom or a $C_1$—$C_5$ alkyl group, and $R^2$ represents a hydrogen atom, a $C_1$—$C_5$ alkyl group or a group represented by $-(CH_2)_n-$Ar, wherein n is 1 or 2, and Ar is an unsubstituted phenyl group or a phenyl group substituted with at least one lower alkoxy group, or a pharmaceutically acceptable acid addition salt thereof, characterized by reacting a compound having the general formula

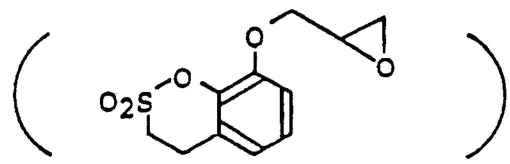

with a compound having the formula $R^1R^2NH$ wherein $R^1$ and $R^2$ have the same meanings as defined above, and, if necessary, further treating the product with a pharmaceutically acceptable acid.

**Patentansprüche**

1. Benzoxathiinderivat der allgemeinen Formel

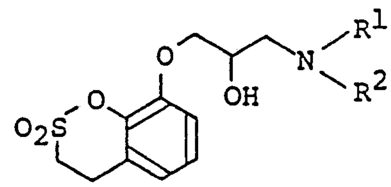

(1)

worin $R^1$ ein Wasserstoffatom oder einen $C_{1-5}$-Alkylgruppe bezeichnet und $R^2$ ein Wasserstoffatom, eine $C_{1-5}$-Alkylgruppe oder eine —$(CH_2)_n$—Ar-Gruppe bezeichnet, worin n 1 oder 2 ist und Ar eine unsubstituierte Phenylgruppe oder eine Phenylgruppe ist, die mit wenigstens einer Niedrigalkoxygruppe substituiert ist, oder ein pharmazeutisch annehmbares Säureadditionssalz davon.

2. Benzoxathiinderivat gemäss Anspruch 1, worin $R^1$ ein Wasserstoffatom oder eine Methylgruppe bezeichnet und $R^2$ eine Methylgruppe, Ethylgruppe, Isopropylgruppe, Isobutylgruppe, t-Butylgruppe, sek-Butylgruppe, n-Propylgruppe oder n-Butylgruppe bezeichnet.

3. Benzoxathiinderivat gemäss Anspruch 1, worin $R^1$ ein Wasserstoffatom oder eine Methylgruppe bezeichnet und $R^2$ eine Phenethylgruppe, eine 3,4-Dimethoxyphenethylgruppe, eine 2,3-Dimethoxyphenethylgruppe oder eine 4-Methoxyphenethylgruppe bezeichnet.

4. Pharmazeutische Zusammensetzung, umfassend ein Benzoxathiinderivat gemäss den vorstehenden Ansprüchen und einen pharmazeutisch annehmbaren Träger.

5. Benzoxathiinderivat gemäss Ansprüchen 1 bis 3 zur Verwendung als Medikament.

6. Benzoxathiinderivat gemäss Ansprüchen 1 bis 3 zur Verwendung für die Behandlung von Erkrankungen des kardiovaskulären Systems.

7. Verwendung eines Benzoxathiinderivates der allgemeinen Formel

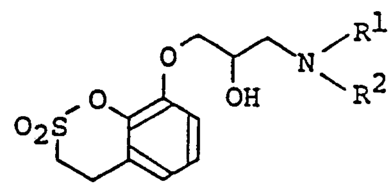

(1)

worin $R^1$ ein Wasserstoffatom oder einen $C_{1-5}$-Alkylgruppe bezeichnet und $R^2$ ein Wasserstoffatom, eine $C_{1-5}$-Alkylgruppe oder eine —$(CH_2)_n$—Ar-Gruppe bezeichnet, worin n 1 oder 2 ist und Ar eine unsubstituierte Phenylgruppe oder eine Phenylgruppe ist, die mit wenigstens einer Niedrigalkoxygruppe substituiert ist, oder ein pharmazeutisch annehmbares Säureadditionssalz davon, zur Herstellung eines Medikamentes für die Behandlung von Erkrankungen des kardiovaskulären Systems.

8. Verfahren zur Herstellung eines Benzoxathiinderivates der allgemeinen Formel

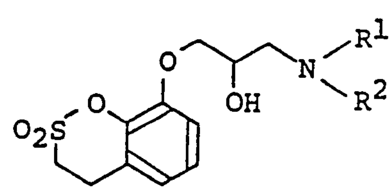

(1)

worin $R^1$ ein Wasserstoffatom oder einen $C_{1-5}$-Alkylgruppe bezeichnet und $R^2$ ein Wasserstoffatom, eine $C_{1-5}$-Alkylgruppe oder eine $-(CH_2)_n-$Ar-Gruppe bezeichnet, worin n 1 oder 2 ist und Ar eine unsubstituierte Phenylgruppe oder eine Phenylgruppe ist, die mit wenigstens einer Niedrigalkoxygruppe substituiert ist, oder ein pharmazeutisch annehmbares Säureadditionssalz davon, dadurch gekennzeichnet, dass man eine Verbindung der allgemeinen Formel

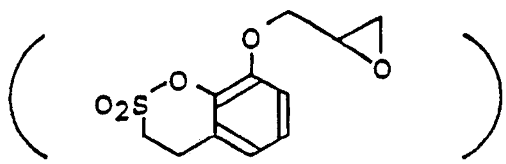

mit einer Verbindung der Formel $R^1R^2NH$ umsetzt, worin $R^1$ und $R^2$ die vorstehend erwähnten Bedeutungen haben, und dass man, wenn erforderlich, das Produkt mit einer pharmazeutisch annehmbaren Säure weiterbehandelt.

## Revendications

1. Dérivé de benzoxathiine de formule générale:

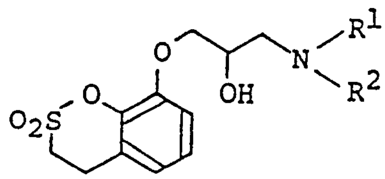

(1)

dans laquelle $R^1$ représente un atome d'hydrogène ou un groupe alkyle en $C_1-C_5$, et $R^2$ représente un atome d'hydrogène, un groupe alkyle en $C_1-C_5$ ou un groupe de formule $-(CH_2)_n-$Ar, n étant égal à 1 ou 2 et Ar représentant un groupe phényle non substitué ou un groupe phényle substitué avec au moins un groupe alkoxy inférieur; ou un sel d'addition avec un acide pharmaceutiquement acceptable de celui-ci.

2. Dérivé de benzoxathiine selon la revendication 1, dans lequel $R^2$ représente un atome d'hydrogène ou un groupe méthyle, et $R^2$ représente un groupe méthyle, éthyle, isopropyle, isobutyle, tert-butyle, sec-butyle, n-propyle ou n-butyle.

3. Dérivé de benzoxathiine selon la revendication 1, dans lequel $R^2$ représente un atome d'hydrogène ou un groupe méthyle, et $R^2$ représente un groupe phénéthyle, 3,4-diméthoxyphénéthyle, 2,3-diméthoxyphénéthyle ou 4-méthoxyphénéthyle.

4. Composition pharmaceutique, comprenant un dérivé de benzoxathiine selon l'une quelconque des revendications précédentes, et un véhicule pharmaceutiquement acceptable.

5. Dérivé de benzoxathiine selon l'une quelconque des revendications 1 à 3, destiné à être employé comme médicament.

6. Dérivé de benzoxathiine selon l'une quelconque des revendications 1 à 3, destiné à être employé pour le traitement de troubles du système cardiovasculaire.

7. Utilisation d'un dérivé de benzoxathiine de formule générale:

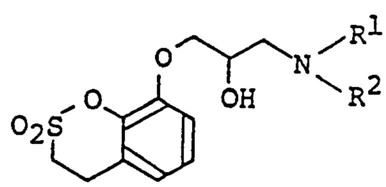

(1)

dans laquelle $R^1$ représente un atome d'hydrogène ou un groupe alkyle en $C_1-C_5$, et $R^2$ représente un atome d'hydrogène, un groupe alkyle en $C_1-C_5$ ou un groupe de formule $-(CH_2)_n-$Ar, n étant égal à 1 ou 2 et Ar représentant un groupe phényle non substitué ou un groupe phényle substitué avec au moins un groupe alkoxy inférieur; ou un sel d'addition avec un acide pharmaceutiquement acceptable de celui-ci, pour la préparation d'un médicament pour le traitement de troubles du système cardiovasculaire.

8. Procédé de préparation d'un dérivé de benzoxathiine de formule générale:

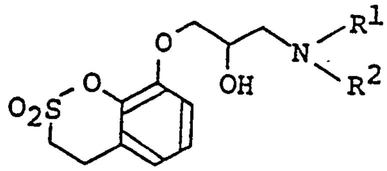

(1)

dans laquelle $R^1$ représente un atome d'hydrogène ou un groupe alkyle en $C_1$—$C_5$, et $R^2$ représente un atome d'hydrogène, un groupe alkyle en $C_1$—$C_5$ ou un groupe de formule $-(CH_2)_n-Ar$, n étant égal à 1 ou 2 et Ar représentant un groupe phényle non substitué ou un groupe phényle substitué avec au moins un groupe alkoxy inférieur; ou un sel d'addition avec un acide pharmaceutiquement acceptable de celui-ci, caractérisé en ce qu'on fait réagir un composé de formule générale:

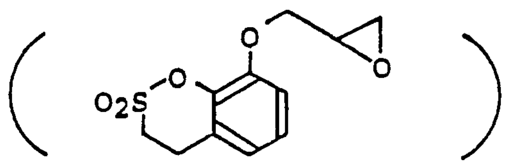

avec un composé de formule $R^1R^2NH$ dans laquelle $R^1$ et $R^2$ ont les mêmes définitions que ci-dessus, et si cela est nécessaire, on traite en outre le produit avec un acide pharmaceutiquement acceptable.